# EUROPÄISCHE PATENTANMELDUNG

(11) **EP 1 891 898 A1**
(43) Veröffentlichungstag der Anmeldung: **27.02.2008**
(21) Anmeldenummer: 06017713.6
(22) Anmeldetag: 25.08.2006
(51) Int. Cl.: A61B 5/15

(54) **Stechgerät**

(71) Anmelder: Roche Diagnostics GmbH, 68305 Mannheim (DE); F. HOFFMANN-LA ROCHE AG, 4070 Basel (CH)
(72) Erfinder: Kraemer, Uwe, 68549 Ilvesheim (DE); List, Hans, 64754 Hesseneck-Kallbach (DE)
(74) Vertreter: Mommer, Niels

(57) **Zusammenfassung**

Die Erfindung betrifft ein Stechgerät zum Erzeugen einer Einstichwunde zum Gewinnen einer Probe einer Körperflüssigkeit, umfassend ein Andruckteil (4) zum Anpressen an ein Körperteil, in dem an einer Probenentnahmestelle eine Einstichwunde erzeugt werden soll, einen Prüfsensor (5) zum Messen eines Wertes eines Prüfparameters, der von einem Zustand des Körperteils an der Entnahmestelle und/oder der Position des Andruckteils (4) relativ zu der Entnahmestelle abhängt, und eine Auswerteeinheit (10), um zu ermitteln, ob der mit dem Prüfsensor (5) gemessene Wert des Prüfparameters einer vorgegebenen Mindestanforderung, die eine erfolgreiche Probengewinnung erwarten lässt, genügt. Erfindungsgemäß ist vorgesehen, dass in einem Betriebsmodus der Wert des Prüfparameters, nachdem er die vorgegebene Mindestanforderung erreicht hat, während eines Wartezeitintervalls mittels des Prüfsensors (5) überwacht wird und die Auswerteeinheit (10) nach Ablauf der Wartezeit einen Einstich auslöst, wenn die während der Wartezeit ermittelten Werte des Prüfparameters in einem Wertebereich liegen, der eine erfolgreiche Probengewinnung erwarten lässt.

## Beschreibung

Die Erfindung betrifft ein Stechgerät zum Erzeugen einer Einstichwunde zum Gewinnen einer Probe einer Körperflüssigkeit, umfassend ein Andruckteil zum Anpressen an ein Körperteil, in dem an einer Probenentnahmestelle eine Einstichwunde erzeugt werden soll, einen Prüfsensor zum Messen eines Wertes eines Prüfparameters, der von einem Zustand des Körperteils an der Probeentnahmestelle und/oder der Position des Andruckteils relativ zu der Entnahmestelle abhängt, und eine an den Prüfsensor angeschlossene Auswerteeinheit zum Auslösen eines Einstichs und zum Ermitteln, ob der mit dem Prüfsensor gemessene Wert des Prüfparameters einer vorgegebenen Mindestanforderung, die eine erfolgreiche Probengewinnung erwarten lässt, genügt.

Nachfolgend wird ohne Beschränkung der Allgemeinheit auf Blut als Beispiel, auch für andere, aus einer Einstichwunde gewinnbare Körperflüssigkeiten, Bezug genommen.

Stechgeräte werden beispielsweise von Diabetikern verwendet, die mehrmals täglich ihren Blutzuckerspiegel überprüfen müssen. Für handelsübliche Stechgeräte werden in der Regel zum einmaligen Gebrauch bestimmte (disposible) Stechelemente verwendet. Derartige Stechelemente können beispielsweise in einem Magazin in ein Stechgerät eingesetzt werden.

Ein ständiges Ziel in der Entwicklung von Stechgeräten besteht darin, mit möglichst geringem Schmerz eine Einstichwunde zu erzeugen, aus der sich eine brauchbare Probe, d.h. eine ausreichende Menge einer Körperflüssigkeit, gewinnen lässt. Sowohl für das Schmerzempfinden als auch für die Probengewinnung ist die Einstichtiefe von großer Bedeutung. Generell gilt, dass das Schmerzempfinden mit zunehmender Einstichtiefe ebenso wie die Flüssigkeitsmenge, die sich aus der Einstichwunde gewinnen lässt, zunehmen. An Stechgeräte wird deshalb die Anforderung gestellt, die Einstichtiefe einerseits so gering wie möglich, andererseits so tief wie nötig zu gestalten.

In diesem Zusammenhang stellt der Anpressdruck, mit dem das Andruckteil an ein Körperteil angepresst wird, einen wichtigen Prüfparameter dar, der von einem Zustand des Körperteils und/oder der Position des Andruckteils relativ zu der Entnahmestelle abhängt. Bei einem zu geringen Anpressdruck kann es dazu kommen, dass bei einem Einstich nicht die gewünschte Einstichtiefe erreicht wird und deshalb keine brauchbare Probe gewonnen werden kann. In einem solchen Fall muss die Einstechprozedur wiederholt werden, was für einen Benutzer äußerst unangenehm ist.

Um das Risiko eines erfolgslosen Einstichs zu reduzieren, sind beispielsweise aus der US 5,879,311 Stechgeräte mit einem Drucksensor bekannt, bei denen automatisch eine Einstichbewegung ausgelöst wird, sobald auf dem Andruckteil ein Druck lastet, der einen vorgegebenen Mindestdruck übersteigt.

Ein Stechgerät mit einem eingebauten Drucksensor wird auch in der EP 1 360 933 A1 beschrieben. Bei diesem Stechgerät wird ein Einstich jedoch nicht automatisch bei Erreichen eines vorgegebenen Mindestdrucks ausgelöst, sondern lediglich angezeigt, ob der auf dem Andruckteil lastende Druck in einem von drei vorgegebenen Bereichen (niedrig, normal und hoch) liegt. Das Auslösen einer Einstichbewegung geschieht durch Drücken einer Taste an dem Gerätegehäuse.

Aufgabe der Erfindung ist es, einen Weg aufzuzeigen, wie mit möglichst großem Benutzerkomfort bei einem Stechgerät das Risiko eines erfolglosen Einstichs, der nicht zur Gewinnung einer brauchbaren Probe führt, reduziert werden kann, ohne dass durch eine unnötige große Einstichtiefe das Schmerzempfinden erhöht wird.

Diese Aufgabe wird mit einem Stechgerät der eingangs genannten Art dadurch gelöst, dass in einem Betriebsmodus die Auswerteeinheit einen Einstich erst auslöst, wenn als notwendige Bedingung eine Wartezeit verstrichen ist, die beginnt, sobald der Wert des Prüfparameters die vorgegebene Mindestbedingung erreicht hat.

Diese notwendige Bedingung kann bei entsprechender Ausgestaltung des Stechgeräts zugleich auch eine hinreichende Bedingung sein, so dass ein Einstich stets nach Ablauf der Wartezeit eingeleitet wird. Möglich ist es auch, dass ein Benutzer einen Einstich durch ein während der Wartezeit vorgenommenes Betätigen eines Bedienungselements verhindern kann. In einem solchen Fall ist das Nicht-Betätigen des entsprechenden Bedienungselementes eine weitere notwendige Bedingung für das Auslösen eines Einstichs.

Bevorzugt hat das Stechgerät eine an die Auswerteeinheit angeschlossene Signaleinheit, um einem Benutzer zu signalisieren, ob der Prüfparameter den vorgegebenen Mindestanforderungen genügt. Diese Maßnahme hat den Vorteil, dass einem Benutzer der Beginn der Wartezeit signalisiert werden kann. Ein Benutzer kann sich somit während der Wartezeit auf den bevorstehenden Einstich einstellen und mental vorbereiten, so dass er nicht von dem Einstich überrascht wird. Bei bekannten Geräten, beispielsweise gemäß US 5,879,311, mit einem automatischen Auslösen eines Einstichs sofort bei Erreichen eines Mindestanpressdrucks, kann ein Benutzer von einem Einstich überrascht werden, da im Voraus nicht bekannt ist, ob der aufbrachte Anpressdruck ausreicht oder nicht.

Bevorzugt ist das Stechgerät derart ausgestaltet, dass der Wert des Prüfparameters, nachdem er die vorgegebene Mindestanforderung erreicht hat, während eines Wartezeitintervalls mittels des Prüfsensors überwacht wird, und die Auswerteeinheit nach Ablauf der Wartezeit einen Einstich genau dann auslöst, wenn die während der Wartezeit ermittelten Werte des Prüfparameters in einem vorgegebenen Wertebereich liegen, der eine erfolgreiche Probengewinnung erwarten lässt.

Bei einem erfindungsgemäßen Stechgerät wird ein Einstich zwar automatisch aber erst nach Ablauf einer vorgegebenen Wartezeit, also mit einer zeitlichen Verzögerung, ausgelöst. Auf diese Weise hat ein Benutzer die Möglichkeit, auch nach dem Anpressen des Andruckteils des Stechgeräts an ein Körperteil, einen Einstich zu verhindern, beispielsweise um das Gerät zu versetzten und eine Probe an einem anderen Körperteil bzw. an einer anderen Entnahmestelle zu entnehmen.

Diesen Vorteil, einen mit dem Ansetzen des Stechgeräts eingeleiteten Probengewinnungsvorgang abzubrechen, bieten ansonsten nur Geräte, bei denen ein Benutzer zum Auslösen eines Einstichs ein Auslöseelement betätigen muss. Das Betätigen eines solchen Auslöseelements kostet viele Benutzer jedoch Überwindung und wird als psychologische Belastung empfunden. Mit einem erfindungsgemäßen Stechgerät können deshalb die an sich widersprüchlichen Vorteile eines automatischen Auslösens mit einer Kontrolle des Benutzers über den Einstichvorgang kombiniert werden.

Bei dem Prüfsensor einer erfindungsgemäßen Vorrichtung handelt es sich bevorzugt um einen Drucksensor, mit dem ermittelt werden kann, ob der Anpressdruck einen vorgegebenen Mindestwert übersteigt. Alternativ oder ergänzend zu dem Anpressdruck können als Prüfparameter beispielsweise auch die Temperatur des Körperteils an der Entnahmestelle oder der Durchblutungszustand gemessen und ausgewertet werden.

Es ist möglich, die Mindestanforderung, deren Erreichen den Beginn des Wartezeitintervalls festlegt, abweichend von dem vorgegebenen Wertebereich zu wählen, in dem die während der Wartezeit ermittelten Werte des Prüfparameters für ein Auslösen eines Einstichs liegen müssen. Beispielsweise kann die Mindestanforderung ein vorgegebener Mindestdruck p1 sein, und der vorgegebene Wertebereich alle Druckwerte oberhalb eines Druckwertes p2 umfassen, wobei der Druckwert p2 kleiner als der Mindestdruckwert p1 ist. Auf diese Weise kann ein unnötiges Abbrechen der Wartezeit vermieden werden, wobei selbstverständlich der Druckwert p2 so zu wählen ist, dass auch bei dem Druck p2 eine erfolgreiche Probengewinnung zu erwarten ist. Bei Verwendung mechanischer Sensoren, beispielsweise einer Schnappscheibe, tritt oft eine Hysterese auf, so dass ein zum Umschnappen der Schnappscheibe erforderlicher Mindestdruckwert p1 automatisch etwas größer als ein Druck p2 ist, bei dessen Unterschreiten die Schnappscheibe in ihrer Ausgangskonfiguration zurückschnappt.

Weitere Einzelheiten und Vorteile der Erfindung werden anhand eines Ausführungsbeispiels unter Bezugnahme auf die beigefügten Zeichnungen erläutert. Die im Folgenden beschriebenen Merkmale des Ausführungsbeispiels können einzeln oder in Kombination zum Gegenstand von Ansprüchen gemacht werden. Es zeigen:
- Fig. 1: ein Ausführungsbeispiel eines erfindungsgemäßen Stechgeräts in einer teilweise geschnittene Ansicht; und
- Fig. 2: das in Fig. 1 dargestellte Ausführungsbeispiel unter Einwirkung eines Anpressdrucks.

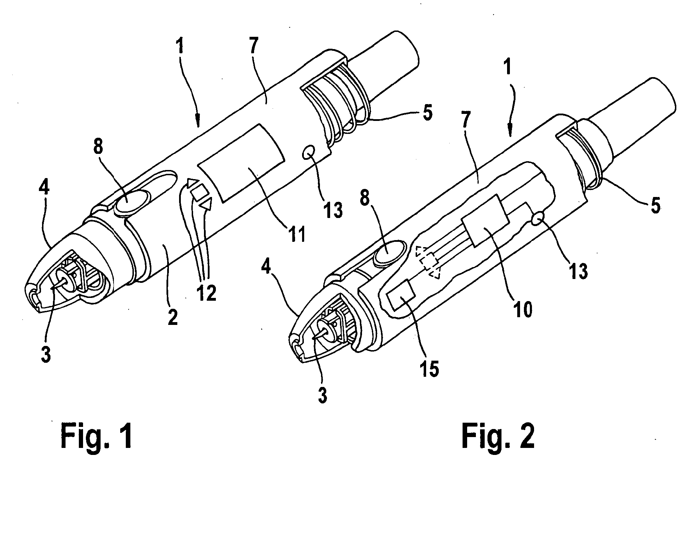

Das in Fig. 1 dargestellte Stechgerät 2 bildet zusammen mit zur einmaligen Verwendung vorgesehenen Lanzetten 3, die in das Stechgerät 2 eingesetzt werden, ein Stechsystem 1. Zum Erzeugen einer Einstichwunde an einer Probenentnahmestelle wird das Stechgerät 2 mit seinem Andruckteil 4 an ein Körperteil eines Benutzers angepresst. Dabei wird das Andruckteil 4 gegenüber dem Gerätegehäuse 7 verschoben. Überschreitet der ausgeübte Anpressdruck einen durch die Stärke einer Feder 5 festgelegten Mindestdruck, wird ein nicht dargestellter Schalter geschlossen und auf diese Weise detektiert, dass das Stechgerät 2 mit einem Druck gegen ein Körperteil eines Benutzers gepresst wird, der eine erfolgreiche Probengewinnung erwarten lässt.

Bei dem dargestellten Ausführungsbeispiel bildet die Feder 5 auf diese Weise einen Prüfsensor und der Anpressdruck bzw. die Information, ob der Anpressdruck einen durch die Stärke der Feder 5 festgelegten Mindestdruck überschreitet, einen Prüfparamter, der von einem Zustand des Körperteils an der Entnahmestelle und/oder der Position des Andruckteils 4 relativ zu der Entnahmestelle abhängt. Anstelle eines Drucksensors, beispielsweise in Form einer Feder 5, können auch andere Prüfsensoren zum Messen eines Wertes eines Prüfparameters verwendet werden, beispielsweise Sensoren zum Messen der Temperatur oder optische Sensoren zur Messung des Durchblutungszustands.

Das dargestellte Stechgerät umfasst ferner eine in Fig. 2 schematisch dargestellte Auswerteeinheit 10, um zu ermitteln, ob der mit dem Prüfsensor gemessene Wert des Prüfparameters einer vorgegebenen Mindestanforderung, die eine erfolgreiche Probengewinnung erwarten lässt, genügt. Bei dem dargestellten Ausführungsbeispiel ist diese Mindestanforderung der zum Komprimieren der Feder 5 erforderliche Mindestpressandruck.

Das dargestellte Messgerät ist in einem Betriebsmodus betreibbar, in dem der Wert des Prüfparameters, nachdem er die vorgegebene Mindestanforderung erreicht hat, während eines Wartezeitintervalls mittels des Prüfsensors 5 überwacht wird und die Auswerteeinheit 10 nach Ablauf der Wartezeit einen Einstich auslöst, wenn alle während der Wartezeit ermittelten Werte des Prüfparameters in einem vorgegebenen Wertebereich liegen, der eine erfolgreiche Probengewinnung erwarten lässt. Bei dem dargestellten Ausführungsbeispiel besteht dieser Wertebereich aus genau den Werten des Prüfparameters, welche die vorgegebene Mindestanforderung erfüllen. Ein Einstich wird also ausgelöst, wenn während der gesamten Wartezeit der Wert des Prüfparameters der vorgegebenen Mindestanforderung genügt hat, d.h. bei dem dargestellten Ausführungsbeispiel der durch die Stärke der Feder 5 festgelegte Mindestanpressdruck nicht unterschritten wurde.

Eine geeignete Auswerteeinheit 10 kann beispielsweise durch einen ASIC (Application Specific Integrated Circuit) oder einen Mikroprozessor realisiert werden. Bei dem dargestellten Ausführungsbeispiel löst die Auswerteeinheit 10 automatisch einen Einstich aus, sobald während einer vorgegebenen Wartezeit von mindestens 0,5 sec., vorzugsweise mindestens 0,8 sec., insbesondere mindestens 1,0 sec., ein durch die Feder 5 vorgegebener Mindestanpressdruck überschritten ist, so dass durch die dadurch bewirkte Verschiebung des Andruckteils 4 gegenüber dem Gerätegehäuse 7 der erwähnte Schalter (nicht dargestellt) geschlossen bleibt. Öffnen dieses Schalters führt dazu, dass die vorgegebene Wartezeit von neuem beginnt. Das Verstreichen der vorgegebenen Wartezeit kann im einfachsten Fall durch ein RC-Glied oder eine Uhr in Form eines getackteten Zählers erfasst werden.

Bei dem dargestellten Ausführungsbeispiel ist die Wartezeit von einem Benutzer einstellbar. Das Stechgerät 2 umfasst zu diesem Zweck eine Anzeigeeinrichtung 11 in Form eines Flüssigkristalldisplays, bevorzugt eines Segmentdisplays, und eine Bedienungseinrichtung mit manuell betätigbaren Bedienungselementen 12. Mittels der Bedienungselemente 12 ist ferner auch die Mindestanforderung, bei dem dargestellten Ausführungsbeispiel also der zum Auslösen erforderliche Mindestanpressdruck, einstellbar, um eine Anpassung des Stechgeräts 1 an die individuellen Bedürfnisse eines Benutzers zu erleichtern.

Sobald der mit dem Prüfsensor 5 gemessene Wert des Prüfparameters den vorgegebenen Mindestanforderungen genügt, wird dies einem Benutzer mittels einer an die Auswerteeinheit 10 angeschlossenen Signaleinheit 13 signalisiert. Bei dem dargestellten Ausführungsbeispiel ist die Signaleinheit 13 eine optische Signaleinheit, die ein Leuchtsignal erzeugt, um den Benutzern zu signalisieren, dass der Prüfparameter den vorgegebenen Mindestanforderungen genügt. Alternativ oder zusätzlich kann beispielsweise auch eine akustische Signaleinheit oder eine Signaleinheit, die eine fühlbare Vibration erzeugt, verwendet werden.

Besonders günstig ist es, dem Benutzer mit der Signaleinheit 13 zusätzlich auch während des Verstreichens der Wartezeit den auftretenden Zeitablauf zu signalisieren. Die Signaleinheit 13 erzeugt deshalb während des Wartezeitintervalls ein Signal, dass mit dem zum Zeitpunkt der Signalerzeugung bereits verstrichenen Anteil des Wartezeitintervalls korreliert, um einen Benutzer den Zeitablauf zu signalisieren. Beispielsweise kann die Leuchtintensität des mit der Signaleinheit 13 erzeugten Signals mit zunehmendem Zeitablauf während des Wartezeitintervalls geändert werden. Auf diese Weise kann ein Benutzer erkennen, wie viel Zeit bis zum automatischen Auslösen eines Einstichs noch verbleibt. Eine weitere Möglichkeit besteht darin, mit der Singaleinheit 13 ein Blinksignal zu erzeugen, dessen Frequenz sich während des Wartezeitintervalls ändert, bevorzugt größer wird. In entsprechender Weise kann auch mit einer akkustischen Signaleinheit oder einer Signaleinheit, die ein taktiles Signal generiert, der Zeitablauf während des Wartezeitintervalls durch eine Änderung der Signalintensität oder der Frequenz angezeigt werden. Insbesondere kann auch die Anzeigeeinrichtung 11 als Signaleinheit genutzt werden, um einem Benutzer zu signalisieren, ob der Prüfparameter den vorgegebenen Mindestanforderungen genügt und welcher Anteil des Wartezeitintervalls bei Signalerzeugung bereits verstrichen ist. Beispielsweise kann auf dem Display ein Balken angezeigt werden, der sich während der Wartezeit verkürzt, so dass ein Einstich ausgelöst wird, sobald der Balken verschwunden ist. Eine andere intuitiv verständliche Darstellungsmöglichkeit ist ein Bild einer Sanduhr, die während der Wartezeit leer läuft.

Mittels der Bedienungselemente 12 kann das Stechgerät 2 in einen weiteren Betriebsmodus versetzt werden, in dem ein Einstich mittels eines benutzerbetätigbaren Auslöseelements 8 auslösbar ist, sobald der Wert des Prüfparameters der vorgegebenen Mindestanforderung genügt. Das benutzerbetätigbare Auslöseelement 8 ist bei dem dargestellten Ausführungsbeispiel eine Taste. Möglich ist es aber auch, das benutzerbetätigbare Auslöseelement 8 dadurch auszubilden, dass beispielsweise eine Erhöhung des Anpressdrucks, mit dem das Andruckteil 4 gegen das Körperteil gepresst wird, und/oder durch einen zusätzlichen Verschiebeweg des Andruckteils 4 gegenüber dem Gerätegehäuse 7 zu realisieren.

Besonders günstig ist es, wenn das benutzerbetätigbare Auslöseelement 8 in diesem weiteren Betriebsmodus durch eine Sicherungseinrichtung (nicht dargestellt) gesperrt ist, bis der Wert des Prüfparameters der vorgegebenen Mindestanforderung genügt. Auf diese Weise kann sichergestellt werden, dass ein Benutzer einen Einstich nur dann Auslösen kann, wenn mit einer erfolgreichen Probengewinnung zu rechnen ist. Eine derartige Sicherungseinrichtung kann beispielsweise in die Auswerteeinrichtung 10 integriert sein.

Einen besonders hohen Benutzerkomfort bieten Stechgeräte 2, mit denen nicht nur eine Einstichwunde erzeugt wird, sondern die zusätzlich eine Messeinrichtung 15 zur Untersuchung einer gewonnenen Körperflüssigkeitsprobe umfassen. Geeignete Messeinrichtungen 15, beispielsweise zur photometrischen oder elektrochemischen Untersuchung, sind in handelsüblichen Analysehandgeräten zur Bestimmung des Blutglukosegehalts enthalten. Beispielsweise können eine Probenaufnahmeeinheit und ein Testfeld zur elektrochemischen oder photometrischen Bestimmung einer Analytkonzentration in das Stechelement 3 integriert sein. Geeignete Stechelemente, die einen Kapillarkanal aufweisen, in dem Körperflüssigkeit während einer Sammelphase eindringen und zu dem Testfeld gelangen kann, sind im Stand der Technik, beispielsweise aus der US 2003/0171699 A1, bekannt, die diesbezüglich durch Bezugnahme zum Gegenstand der Anmeldung gemacht wird. Bei derartigen Stechgeräten macht die Probenaufnahme für den Benutzer keine zusätzlichen Handhabungsschritte erforderlich. Dies ist insbesondere für Benutzer, deren motorische Beweglichkeit durch Alter oder Krankheit eingeschränkt ist, ein wichtiger Vorteil.

## Patentansprüche

1. Stechgerät zum Erzeugen einer Einstichwunde zum Gewinnen einer Probe einer Körperflüssigkeit, umfassend
ein Andruckteil (4) zum Anpressen an ein Körperteil, in dem an einer Probenentnahmestelle eine Einstichwunde erzeugt werden soll,
einen Prüfsensor (5) zum Messen eines Wertes eines Prüfparameters, der von einem Zustand des Körperteils an der Entnahmestelle und/oder der Position des Andruckteils (4) relativ zu der Entnahmestelle abhängt, und
eine Auswerteeinheit (10) zum Auslösen eines Einstichs und zum Ermitteln, ob der mit dem Prüfsensor (5) gemessene Wert des Prüfparameters einer vorgegebenen Mindestanforderung, die eine erfolgreiche Probengewinnung erwarten lässt, genügt,
**dadurch gekennzeichnet, dass**
in einem Betriebsmodus die Auswerteeinheit (10) einen Einstich erst auslöst, wenn als notwendige Bedingung eine Wartezeit verstrichen ist, die beginnt, sobald der Wert des Prüfparameters die vorgegebene Mindestbedingung erreicht hat.

2. Stechgerät nach Anspruch 1, **dadurch gekennzeichnet, dass** der Wert des Prüfparameters, nachdem er die vorgegebene Mindestanforderung erreicht hat, während der Wartezeit mittels des Prüfsensors (5) überwacht wird, und die Auswerteeinheit (10) nach Ablauf der Wartezeit einen Einstich genau dann auslöst, wenn die während der Wartezeit ermittelten Werte des Prüfparameters in einem vorgegebenen Wertebereich liegen, der eine erfolgreiche Probengewinnung erwarten lässt.

3. Stechgerät nach einem der vorhergehenden Ansprüche, **dadurch gekennzeichnet, dass** die Wartezeit mindestens 0,5 s, vorzugsweise mindestens 0,8 s, insbesondere mindestens 1,0 s beträgt.

4. Stechgerät nach einem der vorhergehenden Ansprüche, **gekennzeichnet durch** eine Bedienungseinrichtung (12), mit der die Wartezeit von einem Benutzer einstellbar ist.

5. Stechgerät nach einem der vorhergehenden Ansprüche, **gekennzeichnet durch** eine an die Auswerteeinheit (10) angeschlossene Signaleinheit (13), um einem Benutzer zu signalisieren, ob der Prüfparameter den vorgegebenen Mindestanforderungen genügt.

6. Stechgerät nach Anspruch 5, **dadurch gekennzeichnet, dass** die Signaleinheit (13) während der Wartezeit ein Signal erzeugt, das mit dem zum Zeitpunkt der Signalerzeugung bereits verstrichenen Anteil der Wartezeit korreliert, um einem Benutzer den Zeitablauf zu signalisieren.

7. Stechgerät nach einem der vorhergehenden Ansprüche, **dadurch gekennzeichnet, dass** der Prüfsensor (5) ein Drucksensor ist.

8. Stechgerät nach einem der vorhergehenden Ansprüche, **gekennzeichnet durch** eine Messeinrichtung (15) zur Untersuchung einer Körperflüssigkeitsprobe.

9. Stechgerät nach einem der vorhergehenden Ansprüche, **gekennzeichnet durch** eine Bedienungseinrichtung (12) zum Einstellen der Mindestanforderung an den Prüfparameter.

10. Stechgerät nach einem der vorhergehenden Ansprüche, **dadurch gekennzeichnet, dass** in einem weiteren Betriebsmodus ein Einstich mittels eines benutzerbetätigbaren Auslöseelements (8) auslösbar ist, sobald der Wert des Prüfparameters der vorgegebenen Mindestanforderung genügt.
